# EUROPEAN PATENT APPLICATION

(11) **EP 0 621 052 A2**
(43) Date of publication of application: **26.10.1994**
(21) Application number: 94105373.8
(22) Date of filing: 07.04.1994
(51) Int. Cl.: A61M 16/20

(54) **Variable resistance for the expiratory breathing circuit**

(30) Priority: 19.04.1993 IT MI930763
(71) Applicant: DAR SOCIETA' PER AZIONI, I-41037 Mirandola (Modena) (IT)
(72) Inventor: Damia, Giorgio, I-20154 Milano (IT)
(74) Representative: Modiano, Guido, Dr.-Ing.

(57) **Abstract**

Pressure adjustment valve for circuits for the manual administration of gas mixtures in the medical field, including a body (2) that can be interposed on the circuit of a manual respiration unit and has an intake port (3) and a discharge port (4). Inside the body (2) there is provided an adjustment valve (10,21) that can be positioned so as to vary the useful passage section of the gas stream in order to modify the resistance opposed by the adjustment valve (10,21) to the gas stream.

## Description

The present invention relates to a pressure adjustment valve for circuits for the manual administration of gas mixtures in the medical field.

As it is known, throttling valves having the purpose of adjusting the pressure of the gas stream are currently used in circuits for the manual administration of gas mixtures in the medical field.

These valves, which are normally provided on a connector that mutually connects the manually-operated bag, the fresh-gas feed tube and the patient's connector, are currently constituted by a cylindrical body provided with intake and discharge ports for the required connections.

Pressure adjustment is generally performed on the intake port by means of a disk which is pushed by an adjustable spring so as to act both as check valve and as flow throttling element according to the setting of the spring.

These valves, in addition to being mechanically relatively complicated, are generally difficult to calibrate, due to the fact that calibration is performed by setting a spring, with the unavoidable expected errors; furthermore, this solution, which is based on the use of a moving disk, is liable to jam both due to difficulties in the sliding of the disk and because any secretions of the patient often reach the working area of the disk, with the possible risk of jamming it.

The aim of the present invention is to solve the problems described above by providing a pressure adjustment valve for circuits for the manual administration of gas mixtures in the medical field which allows to adjust the flow without using moving parts and without having to provide elastic calibration means.

Within the scope of the above aim, a particular object of the invention is to provide an adjustment valve that allows to always obtain an extremely precise adjustment that can be easily and quickly visualized outside, thus considerably facilitating its adjustment.

Another object of the present invention is to provide a pressure adjustment valve that, by virtue of its particular constructive characteristics, is capable of giving the greatest assurances of reliability and safety in use.

A further object of the present invention is to provide a pressure adjustment valve for circuits for the manual administration of gas mixtures in the medical field that can be obtained easily starting from commonly commercially available elements and materials and is furthermore competitive from a merely economical point of view.

With this aim, these objects and others in view, there is provided, according to the present invention, a pressure adjustment valve for circuits for the manual administration of gas mixtures in the medical field which comprises a body that can be interposed on the circuit of a manual respiration unit and has an intake port and a discharge port, characterized in that it comprises, inside said body, an adjustment element that can be positioned so as to vary the useful passage section of the gas stream in order to modify the resistance opposed by the adjustment valve to the gas stream.

Further characteristics and advantages of the pressure adjustment valve for circuits for the manual administration of gas mixtures in the medical field according to the present invention, will become apparent from the following detailed description of two preferred but not exclusive embodiments thereof, illustrated only by way of non-limitative example in the accompanying drawings, wherein:
figure 1 is a schematic elevation view of a first embodiment of the adjustment valve;
figure 2 is an exploded sectional view of the adjustment valve;
figure 3 is a sectional view of the adjustment valve;
figure 4 is a sectional view, taken along the plane IV-IV of figure 3;
figure 5 is an elevation view of a second embodiment of the pressure adjustment valve;
figure 6 is a sectional exploded view of the valve;
figure 7 is a sectional view of the valve;
figure 8 is a sectional view, taken along the plane VIII-VIII of figure 7.

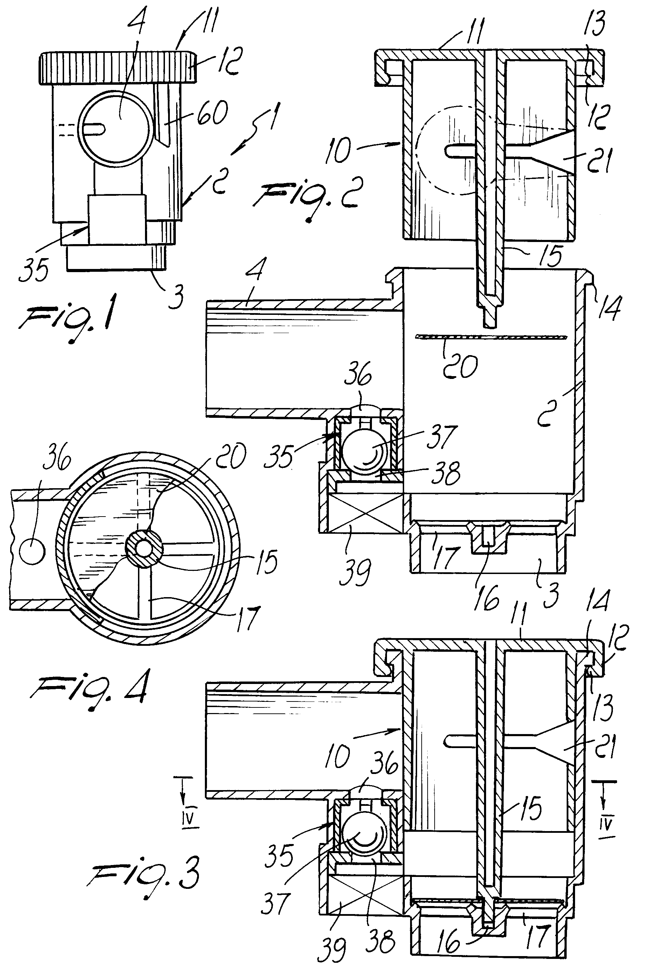

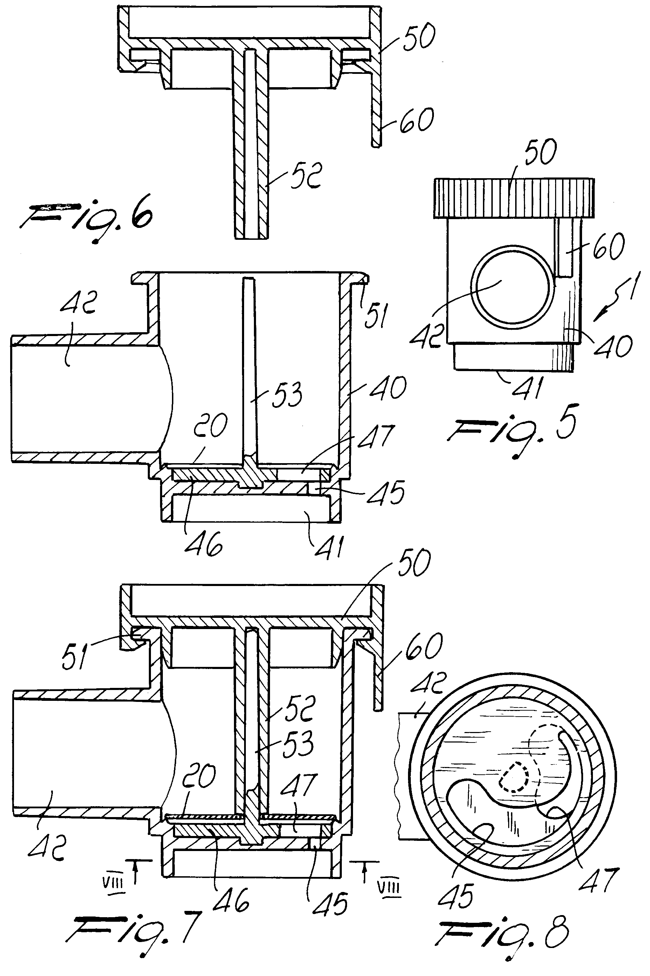

With reference to the above figures and particularly to figures 1 to 4, the pressure adjustment valve for circuits for the manual administration of gas mixtures in the medical field, according to the present invention, generally designated by the reference numeral 1, comprises a cylindrical body 2 having an axial intake port 3 and a discharge port 4 that extends radially from the side wall of the body 2.

Inside said cylindrical body 2 it is possible to arrange an adjustment element advantageously constituted by a cylindrical skirt 10 that enters the body 2 and the outer wall of which is very close to the inner wall. The skirt 10 is closed in an upward region by a lid 11 that has a ridge or rim 12 provided with a tooth 13 for coupling to an annular protrusion 14 formed at the top of the cylindrical body 2, so as to obtain a stable coupling between the skirt 10 and the cylindrical body 2 despite allowing mutual rotation.

An axial stem 15 extends from the bottom 11 of the skirt 10 and enters a centering seat 16 which is supported by a cross-shaped element 17 arranged at the inlet 3.

There is also a membrane 20 resting on the cross-shaped element 17 and in practice forming a check valve.

The particularity of the invention resides in the fact that pressure adjustment is performed by varying the useful passage section, and for this purpose on the lateral surface of the skirt 10 there is, at the discharge port 4, a slot 21 that runs along the circumference of the skirt with a variable cross-section so that, by varying the arrangement of the skirt 10 with respect to the cylindrical body 2, it is possible to vary the useful passage section, thus modifying the resistance of the adjustment valve to the gas stream.

Obviously, the resistance increases if a narrower part of the slot 21 is arranged at the radial discharge port 4 and decreases when a portion of the skirt 10 that has a wider slot 21 is arranged at the discharge port 4.

Furthermore, a bypass valve, generally designated by the reference numeral 35, is provided monolithically with the cylindrical body 2 and intervenes in case of overpressure; said bypass valve is advantageously constituted by a branching port 36 which is provided on the discharge port 4 and below which there is a metal ball 37 retained on the connecting port or opening 38 both by its own weight and by the action of a permanent magnet 39, so that only in case of overpressure can the ball 37 overcome the return action produced by its own weight and by the permanent magnet, freeing the port 38 which allows connection to the inside of the cylindrical body 2 even if, for any reason, the connection provided by means of the discharge port 4 is obstructed.

According to a different embodiment which is conceptually linked to the preceding one and illustrated in figures 5 to 8, there is again a cylindrical body, now designated by the reference numeral 40, having an axial intake port 41 and a radial discharge port 42. An inlet 45 is formed at the intake port 41 and is constituted by a slot defining a cross-section which varies along the circumference.

A disk-like element 46 is rotatably mounted above the wall that forms the inlet 45 and is provided with an opening 47 that varies the useful air passage section according to its arrangement with respect to the slot 45 with circumferentially variable cross-section.

Furthermore, above the disk-like element 46 there is again a membrane 20 that in practice forms a check valve.

The cylindrical body 40 is closed by a plug-like element 50 that couples in a snap-together manner to the upper edge 51 of the cylindrical container 40 and is provided with an axial stem 52 that rigidly rotationally couples to a pivot 53 extending from the disk-like element 46 so as to rotate it.

Furthermore, both the plug-like element 50 and the lid 11 have a notch, designated by the reference numeral 60 for both embodiments, that extends axially and acts as reference element to clearly indicate externally the mutual arrangement of the cylindrical body and of the plug or lid, so as to allow to visualize which passage section is used.

It is thus evident, from what has been described above, that the invention achieves the intended aim and objects, and particularly the fact is stressed that pressure adjustment is performed in this case by varying the useful flow passage sections, i.e. with parts that are static in their operation and are not subject to jammings of any kind and allow to perform an extremely precise adjustment.

Furthermore, the overpressure valve element is also directly integrated in the valve body in the solution illustrated in figures 1 to 4.

The invention thus conceived is susceptible to numerous modifications and variations, all of which are within the scope of the inventive concept.

All the details may furthermore be replaced with other technically equivalent elements.

In practice, the materials employed, as well as the contingent shapes and dimensions, may be any according to the requirements.

Where technical features mentioned in any claim are followed by reference signs, those reference signs have been included for the sole purpose of increasing the intelligibility of the claims and accordingly, such reference signs do not have any limiting effect on the scope of each element identified by way of example by such reference signs.

## Claims

1. Pressure adjustment valve for circuits for the manual administration of gas mixtures in the medical field, comprising a body (2,40) that can be interposed on the circuit of a manual respiration unit and having an intake port (3,41) and a discharge port (4,42), characterized in that it comprises, inside said body (2,40), an adjustment element (10,21,46,47) that can be positioned so as to vary the useful passage section of the gas stream in order to modify the resistance opposed by the adjustment valve (1) to the gas stream.

2. Adjustment valve according to claim 1, characterized in that said adjustment element is constituted by a cylindrical skirt (10) insertable inside said body (2) and having, on its side wall, a slot (21) that runs along its circumference with a variable cross-section at the region affected by said discharge port (4).

3. Adjustment valve according to claim 2, characterized in that said skirt (10) is closed, at the top, by a lid (11) that has a ridge (12) provided with a tooth (13) for coupling to a corresponding annular protrusion (14) formed at the top of said body (2).

4. Adjustment valve according to claim 2, characterized in that an axial stem (15) extends from the bottom of said skirt (10) and can be inserted in a centering seat (16) which is supported by a cross-shaped element (17) located at said intake port (3).

5. Adjustment valve according to claim 4, characterized in that it comprises a check valve that is constituted by a membrane (20) supported by said cross-shaped element (17).

6. Adjustment valve according to claim 1, characterized in that it comprises, monolithically with said body (2), a bypass valve (35) which is constituted by a ball (37) arrangeable on a connecting opening (38) that connects the discharge port (4) to the inside of said body (2), and in that said ball (37) can be retained in position on said connecting opening (38) by virtue of its own weight and by the action of a permanent magnet (39).

7. Adjustment valve according to claim 1, characterized in that said adjustment element is constituted by a disk-like element (46) which has a rotatable opening (47) at an inlet (45) that runs along its circumference with a variable useful section defined at said intake port (41).

8. Adjustment valve according to claim 7, characterized in that it comprises a plug-like element (50) that can be arranged so as to close said body (40) and can be coupled to the upper edge of said body (40), said plug-like element (50) being rigidly rotationally connected to said disk-like element (46).

9. Adjustment valve according to claim 8, characterized in that it comprises a notch (60) extending from said plug-like element (50) and from a lid (11) closing said body (40) to visualize the mutual arrangement of said adjustment element (46) and said body (40).
